Europäisches Patentamt

European Patent Office

Office européen des brevets

(11) Veröffentlichungsnummer : **0 330 647 B1**

(12) **EUROPÄISCHE PATENTSCHRIFT**

(45) Veröffentlichungstag der Patentschrift :
**08.09.93 Patentblatt 93/36**

(51) Int. Cl.[5] : **A61K 35/16**, A61K 37/02,
A61K 39/395, C07K 3/12

(21) Anmeldenummer : **89890044.4**

(22) Anmeldetag : **20.02.89**

(54) **Verwendung von Chymotrypsin zum Unwirksammachen des Präkallikrein-Aktivators.**

Die Akte enthält technische Angaben, die nach dem Eingang der Anmeldung eingereicht wurden und die nicht in dieser Patentschrift enthalten sind.

(30) Priorität : **26.02.88 AT 494/88**

(43) Veröffentlichungstag der Anmeldung :
**30.08.89 Patentblatt 89/35**

(45) Bekanntmachung des Hinweises auf die Patenterteilung :
**08.09.93 Patentblatt 93/36**

(84) Benannte Vertragsstaaten :
**AT BE CH DE ES FR GB IT LI LU NL SE**

(56) Entgegenhaltungen :
**EP-A- 0 120 835**
**FR-A- 2 133 670**

(56) Entgegenhaltungen :
**NEW ENGLAND JOURNAL OF MEDICINE, Band 313, Nr. 9, 1985; M.M.EIBL, Seiten 581 JOINT WHO/IABS SYMPOSIUM ON THE STAN-DARDIZATION OF ALBUMIN; PLASMA SUB-STITUTES AND PLASMAPHERESIS, Geneva (CH), 1980; G.LÜBEN et al.**
**DEVELOP. BIOL. STANDARD, Band 48, Basel (CH), 1981; S.KARGER, Seiten 123-127**

(73) Patentinhaber : **IMMUNO Aktiengesellschaft**
**Industriestrasse 67**
**A-1221 Wien (AT)**

(72) Erfinder : **Linnau, Yendra, Dr.**
**Lavendelweg 24**
**A-1224 Wien (AT)**

(74) Vertreter : **Wolfram, Gustav, Dipl.-Ing.**
**Patentanwälte Sonn, Pawloy, Weinzinger & Wolfram, Riemergasse 14**
**A-1010 Wien (AT)**

EP 0 330 647 B1

## Beschreibung

Die Erfindung betrifft die Herstellung von Blutprodukten aus menschlichem oder tierischem Plasma, insbesondere von Albumin- und Immunglobulin-G-Präparationen, wobei diese Präparationen durch fraktionierte Anreicherung von Plasmaproteinen erhalten werden.

Plasma enthält den Hageman Faktor (Faktor XII), ein Proenzym des Präkallikrein-Aktivators (PKA). Die aktivierten Hageman Faktoren (Faktor XIIa und Faktor XIIf) beeinflussen das Blutgerinnungsschema, das fibrinolytische und das Kallikrein-Kinin-System.

Bei der Herstellung von Blutprodukten aus Plasma können diese Präkallikrein-Aktivatoren gebildet werden, und die hergestellten Präparationen weisen einen PKA-Gehalt auf. Solche Blutprodukte, wie Albumin und Immunglobulin-G-Präparationen, wenn sie in verhältnismäßig großen Mengen infundiert werden, können nachteilige Nebenreaktionen, z.B. Schockzustände, verursachen (vgl. B.M. Alving et al., The New England Journal of Medicine, Vol. 299 (1978), 66).

Ein Verfahren zur Inaktivierung von Unverträglichkeitsreaktionen hervorrufenden Substanzen in Blutprodukten unter Verwendung von immobilisiertem Chymotrypsin ist aus der EP-A - 0 120 835 bekannt. Als Unverträglichkeitsreaktionen wurden aber in erster Linie vasoaktive Nebenerscheinungen und antikomplementäre Aktivität in Betracht gezogen.

Es ist weiters bekannt, PKA zu inaktivieren. So wird in der US-A - 4,251,510 (Tankersley) die Entfernung des PKA durch Adsorption mittels silikahältiger Substanzen beschrieben. Hierbei besteht die Gefahr, daß Schwermetallspuren in die Präparation gelangen. Weiters ist in der AT-B - 376.367 (Philapitsch et al.) ein Zusatz von Inhibitoren, wie $C_1$-Esteraseinhibitor, empfohlen. Derartige Inhibitoren sind schwer zugänglich.

Die Erfindung bezweckt die Vermeidung solcher Schwierigkeiten und stellt sich die Aufgabe, die Herstellung von Blutprodukten zu ermöglichen, die das Risiko von unerwünschten Nebenreaktionen vermeiden, und beruht auf der Erkenntnis, daß Chymotrypsin geeignet ist, unerwünschten Präkallikrein-Aktivator enzymatisch zu spalten und dessen Fähigkeit, Präkallikrein in Kallikrein umzuwandeln, unwirksam zu machen.

Die Erfindung besteht dementsprechend in der Verwendung von Chymotrypsin zum Unwirksammachen des Präkallikrein-Aktivators in Blutprodukten aus menschlichem oder tierischem Plasma, insbesondere in Albumin- und Immunglobulin-G-Präparationen.

Die Erfindung besteht weiters in der Verwendung von Chymotrypsin zum Unwirksammachen des Präkallikrein-Aktivators in Blutprodukten aus menschlichem oder tierischem Plasma, wobei die Blutprodukte Albumin- und Immunoglobin-G-Präparationen sind, welche Präparationen durch fraktionierte Anreicherung von Plasmaproteinen erhalten werden, mit der Maßgabe, daß in einer beliebigen Stufe des Fraktionierungsprozesses eine Chymotrypsin-Lösung oder immobilisiertes Chymotrypsin den Fraktionen zugefügt wird und vor Fertigstellung der Präparationen Chymotrypsin bzw. das immobilisierte Chymotrypsin aus den Präparationen entfernt wird.

Nach einer bevorzugten Ausführungsform besteht die Erfindung in der Verwendung von Chymotrypsin, die dadurch gekennzeichnet ist, daß einer wässerigen Lösung der angereicherten Plasmaproteine Chymotrypsin in Form einer wässerigen Lösung zugefügt wird, die Plasmaproteine mittels eines Proteinfällungsmittels gefällt und damit von der Chymotrypsinlösung getrennt werden, worauf die Plasmaproteine zu den fertigen Präparationen aufgearbeitet werden.

Nach einer anderen bevorzugten Ausführungsform besteht die Erfindung in der Verwendung von Chymotrypsin mit der Maßgabe, daß einer wässerigen Lösung der angereicherten Plasmaproteine an Sepharose immobilisiertes Chymotrypsin zugefügt wird, dann die festen Stoffe zur Abtrennung des Chymotrypsins aus der Lösung entfernt und die die Proteine enthaltenden Lösungen zu den fertigen Präparationen aufgearbeitet werden.

Die Erfindung wird durch folgende Beispiele näher erläutert:

Beispiel 1: Herstellung einer Albuminpräparation

Zu 7 l menschlichen Blutplasmas werden bei einem pH-Wert von 7,2 und einer Temperatur von -2°C 8 % Äthylalkohol zugesetzt, wobei ein Niederschlag ausfällt. Nach Abtrennen des Niederschlages wird die Äthylalkoholkonzentration auf 25 % erhöht und die Temperatur auf -6°C gesenkt. Der ausfallende Niederschlag enthält Immunglobulin, wird abgetrennt und die Äthanolkonzentration des Überstandes wird auf 40 % erhöht, bei einem pH-Wert von 6,5 und einer Temperatur von -7°C. Der gebildete, alpha- und beta-Globulin enthaltende Niederschlag wird abgetrennt und verworfen.

Der pH-Wert des Überstandes wird auf 5,4 gestellt, dabei fällt Albumin aus. Dieses wird durch Zentrifugieren abgetrennt und einem weiteren Reinigungsschritt unterworfen: der Niederschlag wird in Wasser gelöst und die Äthanolkonzentration bei einem pH-Wert von 4,8 und einer Temperatur von -2°C auf 10 % gestellt.

Der Niederschlag wird abgetrennt und verworfen; die Äthylalkoholkonzentration des Überstandes wird auf 40 % erhöht, die Temperatur auf -8°C gesenkt und der pH-Wert auf 5,1 gestellt. Der Albuminniederschlag wird durch Zentrifugieren abgetrennt. Zur Entfernung des Äthanols wird der gelöste Niederschlag einer Ultrafiltration unterworfen und die Albuminlösung sterilfiltriert. Die sterile Albuminlösung wird mit 0,1 Einheit Chymotrypsin (Sigma, Produktnummer: C-4129, Lot 85 F-8130) pro 100 mg Albumin bei +37°C unter Rühren inkubiert: nach 48 h ist die Inaktivierung des PKA beendet.

Zur Entfernung des gelösten Chymotrypsins kann das Albumin mittels 45 % EtOH bei -6°C ausgefällt werden und vom Überstand, in dem das Chymotrypsin enthalten ist, abgetrennt werden. Die Albuminlösung wird sodann zu einer verabreichbaren Präparation aufgearbeitet.

Die Wirkung der erfindungsgemäßen Chymotrypsin-Zugabe ist im folgenden erläutert, wobei zur Bestimmung des Präkallikrein-Aktivators die folgende Methode und die angegebenen Reagentien verwendet werden:

Methode: Aus einer gereinigten Präkallikrein-Präparation (PKK) wird mittels eines Präkallikrein-Aktivators (PKA) Kallikrein (KK) generiert. Das Kallikrein spaltet amidolytisch p-Nitroanilid (pNA) von einem spezifischen chromogenen Substrat ab. Die Konzentration von pNA wird photometrisch bei einer Wellenlänge von 405 nm gemessen.

Reagentien:

| | |
|---|---|
| Puffer I: | 6,06 g TRIS und 23,38 g NaCl werden in ca. 500 ml $H_2O$ dest. gelöst und mit verdünnter HCl auf einen pH-Wert von 8,0 eingestellt und mit $H_2O$ dest. auf 1000 ml aufgefüllt. |
| Puffer II: | 1,81 g TRIS, 1,02 g Imidazol und 6,43 g NaCl werden in ca. 500 ml $H_2O$ dest. gelöst und mit verdünnter HCl auf einen pH-Wert von 7,9 gestellt und mit $H_2O$ dest. auf 1000 ml aufgefüllt. |

Chromogenes Substrat:

S 2302 (Kabi) H-D-Prolyl-L-phenylanyl-L-arginin-p-nitroanilid-dihydrochlorid. Eine 10 m-molare wässerige Lösung wird hergestellt.

25 mg S 2302 in 4,1 ml $H_2O$ dest.

Präkallikrein-Präparation: Die Herstellung der Präparation erfolgt nach einer Vorschrift von Harpel, modifiziert von M.S. Horowitz (New York Blood Center). Dabei wird humanes Citratplasma mit Hilfe einer DEAE-Cellulose behandelt. Die nicht an DEAE-Cellulose gebundene Fraktion enthält das Präkallikrein.

Positive Kontrolle (Standard): Als Standard (= Bezugswert) wird eine Albuminpräparation des Bureau of Biologics (BOB) der Food and Drug Administration, Bethesda, Maryland 20205, USA, verwendet. Diese Präparation enthält einen Präkallikrein-Aktivator. Die Kallikrein-Generation mit diesem BOB-Standard stellt den Bezugswert 1 dar bzw. wird gleich 100 % gesetzt.

Probe: Die Probe wird, wenn erforderlich, gelöst bzw. verdünnt in dem Test eingesetzt.

Test: In einem Wasserbad bei einer Temperatur von 37°C werden in ein Plastikröhrchen

100 µl Präkallikrein-Präparation
50 µl Puffer I
25 µl Probe

pipettiert. Nach einer Inkubationszeit von 15 min bei 37°C werden

300 µl Puffer II
50 µl S 2302-Substrat

pipettiert. Diese Mischung wird in ein auf 37°C temperiertes Photometer eingebracht und die Zunahme der optischen Dichte pro min (ΔOD/min) bei einer Wellenlänge von 405 nm, bei einer Schichtdicke von 10 mm, gemessen.

Die Aktivität einer Probe (ΔOD/min) wird faktoriell - gegenüber dem CBER (Center for Biologics Evaluation and Research)-Standard mit der zahl 1 - bzw. in % vom CBER-Standard ausgedrückt; 1 % vom CBER-Standard ist gleich 1 Internationale Einheit.

Die PKA-Inaktivierung im vorliegenden Beispiel 1 verlief wie folgt:

| | PKA | Gehalt an Albumin (Monomere) |
|---|---|---|
| Anfang | 43 IE/ml | 87,6 % |
| nach 24 h | 4 IE/ml | 87,4 % |
| nach 48 h | 1 IE/ml | 87,0 % |

Beispiel 2: Herstellung einer Albuminpräparation

Die Herstellung der Albuminlösung wird wie in Beispiel 1 durchgeführt; anstelle von gelbstem Chymotrypsin werden 0,25 ml an Sepharose immobilisiertem Chymotrypsin pro 100 mg Albumin verwendet. Nach Beendigung des PKA-Inaktivierungsprozesses wird das wasserunlösliche Chymotrypsin durch Filtrieren entfernt und das Albumin zu einer verabreichbaren Präparation aufgearbeitet.

Das immobilisierte Chymotrypsin wurde wie folgt bereitet:

100 ml Sepharose 4 B-Gel (Pharmacia) werden nach einer Waschung mit 400 ml destilliertem Wasser mit 20 g Bromcyan, die in 10 ml Acetonitril gelöst wurden, bei einem pH-Wert von 11,0 versetzt. Das Reaktionsgemisch wird durch ein Eisbad gekühlt. Nach Entfernung der flüssigen Phase wird das Gel mit 80 mg Chymotrypsin (Sigma), das in 100 ml 0,2 molarem $NaHCO_3$ gelöst wurde, versetzt. Das nicht gebundene Trypsin wird vom Trypsin, das an das Gel gebunden ist, durch Filtrieren getrennt.

Nachdem das Gel-Trypsin mit 100 ml einer 1 molaren Glycinlösung versetzt wurde, wird es gründlich mit 0,2 molarer $NaHCO_3$-Lösung proteinfrei gewaschen. Schließlich wird das Gel-Trypsin in 100 ml 0,9 %iger NaCl-Lösung suspendiert - es ist gebrauchsfertig für die PKA-Inaktivierung.

Die PKA-Inaktivierung verlief wie folgt, wobei die Bestimmung des Präkallikrein-Aktivators wie in Beispiel 1 durchgeführt wurde.

|  | PKA | Gehalt an Albumin (Monomere) |
|---|---|---|
| Anfang | 35 IE/ml | 87,5 % |
| nach 24 h | 2 IE/ml | 86,3 % |
| nach 48 h | 1 IE/ml | 84,8 % |

Beispiel 3: Herstellung einer Immunglobulin G-Präparation

Menschliches Blutplasma wird mit 8 % Äthanol versetzt, bei einem pH-Wert von 7,2 und einer Temperatur von -2°C. Nach Abtrennen des Präzipitates wird die Äthanolkonzentration auf 25 % erhöht und gleichzeitig die Temperatur auf -6°C gesenkt. Der Niederschlag, der Immunglobulin enthält, wird weiter gereinigt durch eine Extraktion mit einem Phosphat-Acetat-Puffer und er wird mit 12 % Äthanol bei einem pH-Wert von 5,3 und einer Temperatur von -2°C versetzt.

Der Niederschlag wird verworfen. Die Äthanolkonzentration des Überstandes wird, bei einem pH-Wert von 7,2 und einer Temperatur von -10°C, auf 25 % erhöht. Das ausgefallene, pastenförmige Immunglobulin wird gesammelt und gelöst; das Äthanol wird durch Ultrafiltration entfernt.

Zur PKA-Inaktivierung wird zu der sterilen Lösung 1 E Chymotrypsin (Sigma, Produktnummer C-4129) pro 100 mg Immunglobulin zugesetzt und bei +37°C unter Rühren behandelt. Nach der Behandlung wurde Immunglobulin mit 40 %igem Äthanol bei -6°C gefällt und damit vom Chymotrypsin abgetrennt. Immunglobulin wurde zu einer verabreichbaren Präparation aufgearbeitet.

Die PKA-Inaktivierung verlief wie folgt:

|  | PKA | Gehalt an Monomere IgG |
|---|---|---|
| Anfang | 323 IE/ml | 88,5 % |
| nach 24 h | 4 IE/ml | 87,0 % |
| nach 48 h | 0 IE/ml | 84,6 % |

Beispiel 4: Herstellung einer Immunglobulin G-Präparation

Die Gewinnung der Immunglobulin-hältigen Fraktion erfolgt in gleicher Weise wie in Beispiel 3.

Die Inkubation wird mit dem immobilisierten Chymotrypsin durchgeführt. Zu 1000 mg Protein werden 1,0 ml immobilisiertes Chymotrypsin zugesetzt.

Die PKA-Inaktivierung verlief wie folgt:

4

|          | PKA        | Gehalt an Monomere IgG |
|----------|------------|------------------------|
| Anfang   | 315 IE/ml  | 88,6 %                 |
| nach 24 h| 22 IE/ml   | 87,9 %                 |
| nach 48 h| 0 IE/ml    | 85,8 %                 |

**Patentansprüche**

1. Verwendung von Chymotrypsin zum Unwirksammachen des Präkallikrein-Aktivators in Blutprodukten aus menschlichem oder tierischem Plasma, insbesondere in Albumin- und Immunglobulin-G-Präparationen.

2. Verwendung von Chymotrypsin nach Anspruch 1, dadurch gekennzeichnet, daß die Blutprodukte Albumin- und Immunglobulin-G-Präparationen sind, wobei diese Präparationen durch fraktionierte Anreicherung von Plasmaproteinen erhalten werden, mit der Maßgabe, daß in einer beliebigen Stufe des Fraktionierungsprozesses eine Chymotrypsin-Lösung oder immobilisiertes Chymotrypsin den Fraktionen zugefügt wird und vor Fertigstellung der Präparationen Chymotrypsin bzw. das immobilisierte Chymotrypsin aus den Präparationen entfernt wird.

3. Verwendung von Chymotrypsin nach Anspruch 2, dadurch gekennzeichnet, daß einer wässerigen Lösung der angereicherten Plasmaproteine Chymotrypsin in Form einer wässerigen Lösung zugefügt wird, die Plasmaproteine mittels eines Proteinfällungsmittels gefällt und damit von der Chymotrypsinlösung getrennt werden, worauf die Plasmaproteine zu den fertigen Präparationen aufgearbeitet werden.

4. Verwendung von Chymotrypsin nach Anspruch 2, dadurch gekennzeichnet, daß einer wässerigen Lösung der angereicherten Plasmaproteine an Sepharose immobilisiertes Chymotrypsin zugefügt wird, dann die festen Stoffe zur Abtrennung des Chymotrypsins aus der Lösung entfernt und die die Proteine enthaltenden Lösungen zu den fertigen Präparationen aufgearbeitet werden.

**Claims**

1. The use of chymotrypsin for the inactivation of prekallikrein activator in blood products from human or animal plasma, in particular in albumin and immunoglobulin-G preparations.

2. The use of chymotrypsin according to claim 1, characterised in that the blood products are albumin preparations and immunoglobulin G preparations, these preparations being obtained by a fractionated enrichment of plasma proteins, with the proviso that a chymotrypsin solution or immobilized chymotrypsin is added to the fractions at any stage of the fractionation process and that the chymotrypsin or the immobilized chymotrypsin, respectively, is removed from the preparations before the preparations are completed.

3. The use of chymotrypsin according to claim 2, characterised in that chymotrypsin in the form of an aqueous solution is admixed to an aqueous solution of the enriched plasma proteins, the plamsa proteins are precipitated by means of a protein precipitating agent and thereby are separated from the chymotrypsin solution, whereupon the plasma proteins are processed to finished preparations.

4. The use of chymotrypsin according to claim 2, characterised in that chymotrypsin immobilized on sepharose is added to an aqueous solution of the enriched plasma proteins, subsequently the solid substances are removed from the solution to separate the chymotrypsin and the protein-containing solutions are processed to finished preparations.

**Revendications**

1. Utilisation de chymotrypsine pour l'inactivation de l'activateur de prékallikréine dans des produits sanguins issus de plasma humain ou animal, en particulier dans des préparations d'albumine et d'immunoglobuline G.

2. Utilisation de chymotrypsine selon la revendication 1, caractérisée en ce que les produits sanguins sont des préparations d'albumine et d'immunoglobuline G, ces préparations étant obtenues par concentration fractionnée de protéines plasmatiques, sous réserve que, à un stade quelconque du procédé de fractionnement, on ajoute aux fractions une solution de chymotrypsine ou de la chymotrypsine immobilisée, et que, avant l'achèvement des préparations, on élimine la chymotrypsine ou la chymotrypsine immobilisée des préparations.

3. Utilisation de chymotrypsine selon la revendication 2, caractérisée en ce que l'on ajoute de la chymotrypsine sous forme d'une solution aqueuse à une solution aqueuse des protéines plasmatiques concentrées, on précipite les protéines plasmatiques à l'aide d'un agent de précipitation des protéines et on les sépare ainsi de la solution de chymotrypsine, après quoi on termine le traitement des protéines plasmatiques pour former les préparations finales.

4. Utilisation de chymotrypsine selon la revendication 2, caractérisée en ce que l'on ajoute de la chymotrypsine immobilisée sur du Sepharose à une solution aqueuse des protéines plasmatiques concentrées, on élimine ensuite les substances solides pour séparer la chymotrypsine de la solution et on termine le traitement des solutions contenant les protéines pour former les préparations finales.